# EUROPEAN PATENT APPLICATION

(11) **EP 3 903 576 A1**
(43) Date of publication of application: **03.11.2021**
(21) Application number: 19902993.5
(22) Date of filing: 25.12.2019
(51) Int. Cl.: A01H 3/04, A01N 37/34, A01P 21/00, C07D 271/10, A01G 7/06, A01N 43/824

(54) **JASMONIC ACID ENDOGENY PROMOTING AGENT, AND METHOD FOR PROMOTING JASMONIC ACID ENDOGENY**

(30) Priority: 25.12.2018 JP 2018241785
(71) Applicant: Tokyo University of Science Foundation, Tokyo 162-8601 (JP)
(72) Inventor: KUCHITSU, Kazuyuki, Tokyo 162-8601 (JP); KITAHATA, Nobutaka, Tokyo 162-8601 (JP); SAITO, Yuho, Tokyo 162-8601 (JP); KURAMOCHI, Koji, Tokyo 162-8601 (JP)
(74) Representative: Isarpatent
(86) International application number: PCT/JP2019/050992
(87) International publication number: WO 2020/138228

(57) **Abstract**

Provided are a jasmonic acid endogeny promoting agent and a method for promoting jasmonic acid endogeny using the same, said agent containing a compound shown in formula (1) or a salt thereof as an active ingredient. In formula (1), R1 and R2 independently represent a monovalent organic group, cyano group, halogen atom or the like, and R1, of which there are n instances, contains at least a cyano group or halogen atom, n represents an integer between 1 and 5, and m represents an integer between 0 and 5.

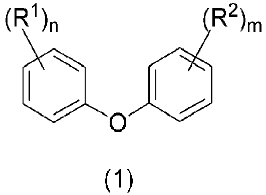

SEQUENCE LISTING

<110>  TOKYO UNIVERSITY OF SCIENCE FOUNDATION

<120>  Agent and method for promoting biosynthesis of jasmonic acid

<130>  RKF-059PCT

<150>  JP 2018-241785

<151>  2018-12-25

<160>  4

<210>  1
<211>  20
<212>  DNA
<213>  Artificial sequence

<220>
<223>  Forward primer for amplifying PDF1.2 gene

<400>  1
tcatggctaa gtttgcttcc                                                    20


<210>  2
<211>  20
<212>  DNA
<213>  Artificial sequence

<220>
<223>  Reverse primer for amplifying PDF1.2 gene

<400>  2
aatacacacg atttagcacc                                                    20


<210>  3
<211>  23
<212>  DNA
<213>  Artificial sequence

<220>
<223>  Forward primer for amplifying EF-1 alpha gene

<400>  3
tgagcacgct cttcttgctt tca                                                23


<210>  4
<211>  24
<212>  DNA
<213>  Artificial sequence

<220>
<223>  Reverse primer for amplifying EF-1 alpha gene

<400>  4
ggtggtggca tccatcttgt taca                                               24

## Description

### TECHNICAL FIELD

The present invention relates to an agent for promoting endogenous jasmonic acid production and a method for promoting endogenous jasmonic acid production.

### BACKGROUND ART

Salicylic acid and jasmonic acid are known as phytohormones that play a role in growth and protective immune responses and the like of plants. In the past, various agrochemical components that activate the salicylic acid pathway and/or jasmonic acid pathway have been proposed.

For example, probenazole (3-(2-propenoxy)-1,2-benzothiazole 1,1-dioxide) has been developed as an agrochemical component that activates the salicylic acid pathway in plants and is widely used for the control of rice blast and other purposes. Probenazole has the effect of promoting the endogenous salicylic acid production in plants, and does not have salicylic acid-like effects by itself. Thus, probenazole has the advantage of a low environmental burden and a low risk of the emergence of drug-resistant bacteria.

On the other hand, jasmonic acid analogs such as prohydrojasmone have been developed as an agrochemical component that activates the jasmonic acid pathway (see, for example, Patent Document 1), and are widely used for the purpose of modulating growth of plants and the like.

Patent Document 1: PCT International Publication No. WO94/18833

### DISCLOSURE OF THE INVENTION

### Problems to be Solved by the Invention

However, the jasmonic acid analogs themselves have jasmonic acid-like effects, and hence they have a large environmental burden and also a risk of adverse effects on the growth of the plants due to an excessive response. Therefore, the development of agrochemical components that promote endogenous jasmonic acid production in plants has been desired, but such an agrochemical component has not been known to date.

The present invention is proposed in view of the background described above, and it is an object of the present invention to provide a novel agent for promoting endogenous jasmonic acid production and a novel method for promoting endogenous jasmonic acid production that promote the endogenous jasmonic acid production in plants.

### Means for Solving the Problems

Specific means for solving the above problems include the following embodiments.

A first aspect of the present invention relates to an agent for promoting endogenous jasmonic acid production containing a compound represented by the following formula (1) or a salt thereof as an active ingredient, 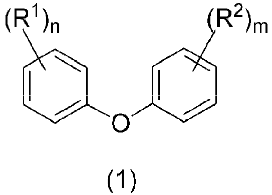
wherein in the formula (1), R¹ and R² represent, each independently, a monovalent organic group, a cyano group, a halogen atom, a hydroxy group, an amino group, a nitro group, a nitroxy group, a mercapto group, a cyanate group, a thiocyanate group, an isothiocyanate group, a sulfo group, a sulfamino group, a sulfino group, a sulfamoyl group, a phospho group, a phosphono group, or a boronyl group, wherein n R¹ include at least a cyano group or a halogen atom;
n represents an integer of 1 to 5,
wherein in the case where n is 2 or more, n R¹ may be identical to or different from one another, and wherein two or more R¹ bonded to adjacent carbon atoms optionally bond to one another to form a ring structure; and
m represents an integer of 0 to 5,
wherein in the case where m is 2 or more, m R² may be identical to or different from one another, and wherein two or more R² bonded to adjacent carbon atoms optionally bond to one another to form a ring structure.

A second aspect of the present invention relates to the agent for promoting endogenous jasmonic acid production according to the first aspect, wherein n R¹ include at least a cyano group.

A third aspect of the present invention relates to the agent for promoting endogenous jasmonic acid production according to the first or second aspect, wherein n is 2 or more.

A fourth aspect of the present invention relates to the agent for promoting endogenous jasmonic acid production according to the third aspect, wherein n R¹ include at least a cyano group and a halogen atom.

A fifth aspect of the present invention relates to the agent for promoting endogenous jasmonic acid production according to the fourth aspect, wherein the compound represented by the formula (1) or a salt thereof is a compound represented by the following formula (2): 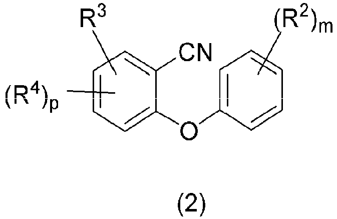
wherein in the formula (2), R³ represents a halogen atom; R⁴ represents a monovalent organic group, a cyano group, a halogen atom, a hydroxy group, an amino group, a nitro group, a nitroxy group, a mercapto group, a cyanate group, a thiocyanate group, an isothiocyanate group, a sulfo group, a sulfamino group, a sulfino group, a sulfamoyl group, a phospho group, a phosphono group, or a boronyl group;
p represents an integer of 0 to 3,
wherein in the case where p is 2 or more, p R⁴ may be identical to or different from one another, and wherein two or more R⁴ bonded to adjacent carbon atoms optionally bond to one another to form a ring structure; and
R² and m are as defined in the formula (1), or a salt thereof.

A sixth aspect of the present invention relates to the agent for promoting endogenous jasmonic acid production according to the fifth aspect, wherein the compound represented by the formula (2) or a salt thereof is a compound represented by the following formula (3-1) or (3-2): 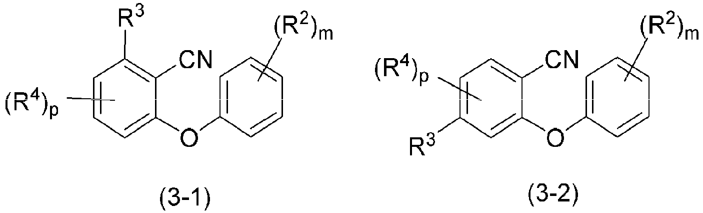 wherein in the formulas (3-1) and (3-2), R², R³, R⁴, m, and p are as defined in the formula (2), or a salt thereof.

A seventh aspect of the present invention relates to a method for promoting endogenous jasmonic acid production, including contacting the agent for promoting endogenous jasmonic acid production according to any one of the first to sixth aspects with a plant.

### Effects of the Invention

The present invention can provide a novel agent for promoting endogenous jasmonic acid production and a novel method for promoting endogenous jasmonic acid production that promote the endogenous jasmonic acid production in plants.

### BRIEF DESCRIPTION OF THE DRAWINGS

FIG. 1 is a graph showing amounts of endogenous jasmonic acid production in *Arabidopsis thaliana* contacted with each of compounds E1, E2 and E3, and comparative compounds C1 and C2;
FIG. 2A is a graph showing an expression level of PDF1.2 gene in *Arabidopsis thaliana* contacted with compound E1;
FIG. 2B is a graph showing an expression level of PDF1.2 gene in *Arabidopsis thaliana* contacted with compound E2;
FIG. 3 is a graph showing an expression level of PDF1.2 gene in *Arabidopsis thaliana* contacted with each of compounds E1, E4 and E5;
FIG. 4 is a graph showing an expression level of PDF1.2 gene in *Arabidopsis thaliana* contacted with each of compounds E1 and E6; and
FIG. 5 is a graph showing the viability of MRC-5 cells when compound E1 or SN-38, an active metabolite of the anticancer drug irinotecan, is added.

### PREFERRED MODE FOR CARRYING OUT THE INVENTION

### <Agent for Promoting Endogenous Jasmonic Acid Production>

An agent for promoting endogenous jasmonic acid production according to the present embodiment contains a compound represented by the following formula (1) or a salt thereof as an active ingredient.

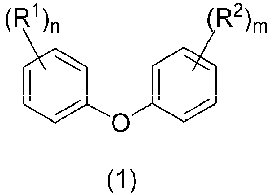

In the formula (1), R¹ and R² represent, each independently, a monovalent organic group, a cyano group, a halogen atom, a hydroxy group, an amino group, a nitro group, a nitroxy group, a mercapto group, a cyanate group, a thiocyanate group, an isothiocyanate group, a sulfo group, a sulfamino group, a sulfino group, a sulfamoyl group, a phospho group, a phosphono group, or a boronyl group, wherein n R¹ include at least a cyano group or a halogen atom.

Examples of the monovalent organic group represented by R¹ or R² include an alkyl group, a cycloalkyl group, an alkenyl group, an alkynyl group, an aryl group, a non-aromatic heterocyclic group, a heteroaryl group, an arylalkyl group, a carboxy group, a formyl group, a formyloxy group, an acetalized formyl group, a formamide group, an isocyanate group, a carbamoyl group; groups represented by -OR, -C(=O)R, -C(=O)OR, -OC(=O)R, -NHR, -NR₂, -C(=NR)R, -C(=NR)NHR, - C(=NR)NR₂, -SR, -C(=O)NHR, -C(=O)NR₂, -C(=N-OR)R, -C(=NR)OR, - OC(=NR)R, -OC(=O)NHR, -OC(=O)NR₂, -NHC(=O)R, -NRC(=O)R, - NHC(=O)OR, -NRC(=O)OR, -NHC(=O)NHR, -NRC(=O)NHR, -NHC(=O)NR₂, - NRC(=O)NR₂, -NHC(=NR)R, -NRC(=NR)R, -S(=O)₂R, -S(=O)₂OR, - OS(=O)₂R, -NHS(=O)₂R, -NRS(=O)₂R, -NHC(=S)NR₂, -NHC(=S)NR₂, - NHC(=S)NHR, -NHC(=S)NHR, -S(=O)₂NHR, -S(=O)₂NR₂, -P(=O)R₂, - P(=O)(OR)₂, -P(=O)(NR₂)₂, -P(=O)(NHR)₂, or -SiR₃ (wherein in the formulas, R represents, each independently, an alkyl group, a cycloalkyl group, an alkenyl group, an alkynyl group, an aryl group, a non-aromatic heterocyclic group, or a heteroaryl group); and the like.

Examples of the alkyl group include linear or branched alkyl groups having 1 to 20 carbon atoms, preferably having 1 to 10 carbon atoms, such as a methyl group, an ethyl group, a propyl group, an isopropyl group, a butyl group, a sec-butyl group, an isobutyl group, a tert-butyl group, a pentyl group, an isopentyl group, and a hexyl group.

Examples of the cycloalkyl group include cycloalkyl groups having 3 to 20 carbon atoms, preferably having 3 to 10 carbon atoms, such as a cyclopropyl group, a cyclobutyl group, a cyclopentyl group, a cyclohexyl group, a bicyclo[2.2.1]heptyl group, a bicyclo[3.2.1]octyl group, and a tricyclo[2.2.1.0]heptyl group.

Examples of the alkenyl group include linear or branched alkenyl groups having 2 to 20 carbon atoms, preferably having 2 to 10 carbon atoms, such as a vinyl group, an allyl group, a propenyl group, an isopropenyl group, a butenyl group, an isobutenyl group, a 1,3-butadienyl group, a pentenyl group, and a hexenyl group.

Examples of the alkynyl group include linear or branched alkynyl groups having 2 to 20 carbon atoms, preferably having 2 to 10 carbon atoms, such as an ethynyl group, a propynyl group, a butynyl group, a pentynyl group, and a hexynyl group.

Examples of the aryl group include monocyclic groups or fused polycyclic groups formed of 2 to 4 rings, such as a phenyl group, a naphthyl group, an anthryl group, a phenanthryl group, a pyrenyl group, a fluorenyl group, an indenyl group, an acenaphthylenyl group, an indanyl group, and an acenaphthenyl group.

Examples of the non-aromatic heterocyclic group include: monocyclic nitrogen-containing groups such as an azetidinyl group, a pyrrolidinyl group, a pyrrolinyl group, a piperidyl group, a tetrahydropyridyl group, a homopiperidinyl group, an octahydroazocinyl group, an imidazolidinyl group, an imidazolinyl group, a pyrazolidinyl group, a pyrazolinyl group, a piperazinyl group, a homopiperazinyl group, and a 2-piperazinonyl group; monocyclic oxygen-containing groups such as a tetrahydrofuranyl group, a tetrahydropyranyl group, and a pyranyl group; monocyclic nitrogen-and-oxygen-containing groups such as a morpholinyl group; monocyclic nitrogen-and-sulfur containing groups such as a thiomorpholinyl group; bicyclic oxygen-containing groups such as an indolinyl group, an isoindolinyl group, a tetrahydroquinolinyl group, a tetrahydroisoquinolinyl group, a 2,3-dihydrobenzofuranyl group, a chromanyl group, a chromenyl group, an isochromanyl group, a 1,3-benzodioxolyl group, a 1,3-benzodioxanyl group, and a 1,4-benzodioxanyl group; bicyclic sulfur-containing groups such as a 2,3-dihydrobenzothienyl group; bicyclic nitrogen-and-oxygen-containing groups such as a benzomorpholinyl group, a dihydropyranopyridyl group, a dihydrodioxynopyridyl group, and a dihydropyridooxazinyl group; heterocyclic spiro ring groups such as a 2-azaspiro[3.3]octyl group, a 2-oxaspiro[3.3]octyl group, a 6-aza-2-oxaspiro[3.3]octyl group, a 1-azaspiro[4.5]decyl group, and a 1-oxaspiro[4.5]decyl group; and the like.

Examples of the heteroaryl group include: monocyclic nitrogen-containing groups such as a pyrrolyl group, a pyridyl group, an imidazolyl group, a pyrazolyl group, a pyrazinyl group, a pyridazinyl group, a pyrimidinyl group, a triazolyl group, and a tetrazolyl group; monocyclic oxygen-containing groups such as a furanyl group; monocyclic sulfur-containing groups such as thienyl; monocyclic nitrogen-and-oxygen-containing groups such as an oxazolyl group, an isoxazolyl group, and an oxadiazolyl group; monocyclic nitrogen-and-sulfur-containing groups such as a thiazolyl group, an isothiazolyl group, and a thiadiazolyl group; bicyclic nitrogen-containing groups such as an indolyl group, an isoindolyl group, a benzoimidazolyl group, an indazolyl group, a benzotriazolyl group, a tetrahydroquinolyl group, a quinolyl group, a tetrahydroisoquinolyl group, an isoquinolyl group, a quinolizinyl group, a cinnolinyl group, a phthalzinyl group, a quinazolinyl group, a quinoxalinyl group, a naphthyridinyl group, a pyrrolopyridyl group, an imidazopyridyl group, a pyrazolopyridyl group, a pyridopyrazyl group, a purinyl group, and a pteridinyl group; bicyclic oxygen-containing groups such as a benzofuranyl group, and an isobenzofuranyl group; bicyclic sulfur-containing groups such as a benzothienyl group; bicyclic nitrogen-and-oxygen-containing groups such as a benzoxazolyl group, a benzoisoxazolyl group, and a benzooxadiazolyl group; bicyclic nitrogen-and-sulfur-containing groups such as a benzothiazolyl group, a benzoisothiazolyl group, a benzothiadiazolyl group, and a thiazolopyridyl group; and the like.

Examples of the arylalkyl group include a benzyl group, a diphenylmethyl group, a trityl group, a phenethyl group, a naphthylmethyl group, and the like.

Examples of the acetalized formyl group include an ethylene-acetalized formyl group, a propylene-acetalized formyl group, and the like.

The monovalent organic group represented by R¹ or R² may be a group formed by bonding one or more types of the organic groups exemplified above. In addition, the monovalent organic group represented by R¹ or R² may be substituted as appropriate with one or more types of groups such as a cyano group, a halogen atom, a hydroxy group, an amino group, a nitro group, a nitroxy group, a mercapto group, a cyanate group, a thiocyanate group, an isothiocyanate group, a sulfo group, a sulfamino group, a sulfino group, a sulfamoyl group, a phospho group, a phosphono group, or a boronyl group.

Examples of the halogen atom represented by R¹ or R² include a fluorine atom, a chlorine atom, a bromine atom, an iodine atom, and the like.

In the formula (1), n represents an integer of 1 to 5. In the case where n is 2 or more, n R¹ may be identical to or different from one another, and two or more R¹ bonded to adjacent carbon atoms optionally bond to one another to form a ring structure. n is preferably 1 to 3, more preferably 1 or 2, and still more preferably 2. In the case where n is 1, R¹ is preferably a cyano group. In the case where n is 2 or more, n R¹ include at least a cyano group, and more preferably include at least a cyano group and a halogen atom. In this case, the halogen atom is preferably a fluorine atom or a chlorine atom, and more preferably a fluorine atom.

In the formula (1), m represents an integer of 0 to 5. In the case where m is 2 or more, m R² may be identical to or different from one another, and two or more R² bonded to adjacent carbon atoms optionally bond to one another to form a ring structure. m is preferably 0 to 2, more preferably 0 or 1, and still more preferably 1. m R² preferably include at least one type of group selected from the group consisting of a C1-C4 alkoxy group, a heteroaryl group, a halogen atom, and amino group.

Among the compounds represented by the formula (1), a compound represented by the following formula (2) is preferable, and a compound represented by the following formula (3-1) or (3-2) is more preferable.

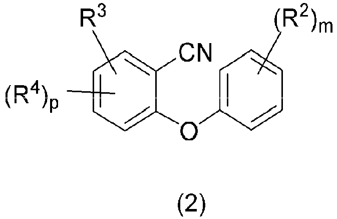

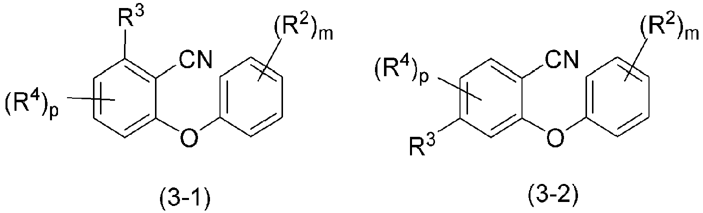

In the formulas (2), (3-1) and (3-2), R³ represents a halogen atom, and R⁴ represents a monovalent organic group, a cyano group, a halogen atom, a hydroxy group, an amino group, a nitro group, a nitroxy group, a mercapto group, a cyanate group, a thiocyanate group, an isothiocyanate group, a sulfo group, a sulfamino group, a sulfino group, a sulfamoyl group, a phospho group, a phosphono group, or a boronyl group. Examples of the halogen atom and the monovalent organic group include groups exemplified for R¹ and R².

In the formulas (2), (3-1) and (3-2), p represents an integer of 0 to 3. In the case where p is 2 or more, p R⁴ may be identical to or different from one another, and two or more R⁴ bonded to adjacent carbon atoms optionally bond to one another to form a ring structure. p is preferably 0 or 1, and more preferably 0.

In the formulas (2), (3-1) and (3-2), R² and m are as defined in the formula (1). In the case where m is 1, the substitution position of R² is not particularly limited, but is preferably at the 4-position when the carbon atom to which the oxygen atom of the ether bond is bonded is assigned to the 1-position.

In the case where the compound represented by the formula (1) has an acidic functional group or a basic functional group, the compound may be in the form of an agrochemically acceptable salt. For example, in the case where the compound represented by the formula (1) has an acidic functional group, the compound may be in the form of an alkali metal salt (sodium salt, potassium salt, or the like), an alkaline earth metal salt (calcium salt, magnesium salt, or the like), an ammonium salt, or the like. Alternatively, in the case where the compound represented by the formula (1) has a basic functional group, the compound may be in the form of a salt with an inorganic acid such as hydrochloric acid, hydrobromic acid, nitric acid, sulfuric acid, phosphoric acid or the like, or may be in the form of a salt with an organic acid such as acetic acid, phthalic acid, fumaric acid, oxalic acid, tartaric acid, maleic acid, citric acid, succinic acid, methanesulfonic acid, benzenesulfonic acid, or p-toluenesulphonic acid.

Specific examples of the compound represented by the formula (1) include the following compounds. However, the compound represented by the formula (1) is not limited to these specific examples.

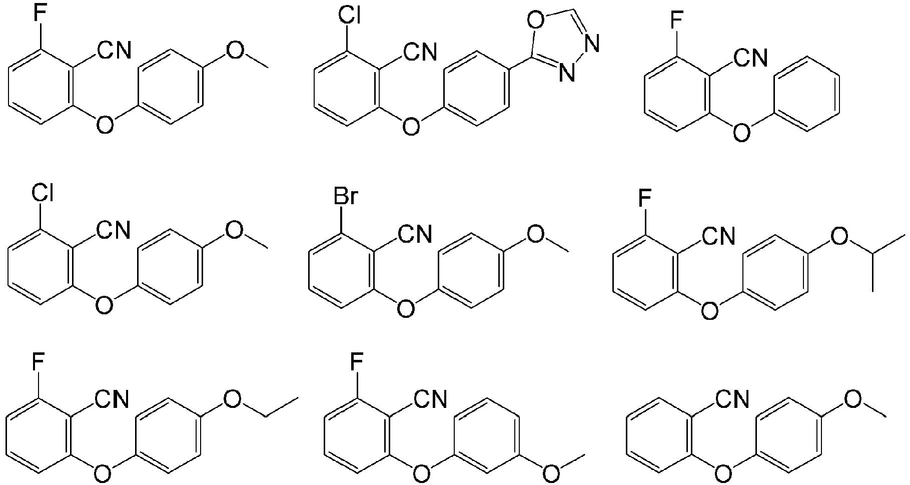

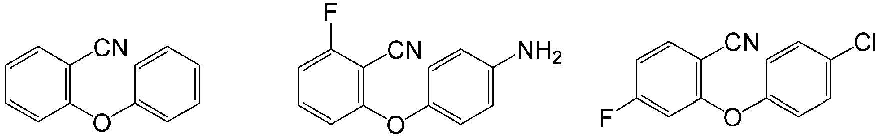

The compound represented by the formula (1) or a salt thereof does not have jasmonic acid-like effects by itself, but has the effect of promoting the endogenous jasmonic acid production in plants.

Jasmonic acid is known to have resistance-inducing effects in plants. Therefore, the agent for promoting endogenous jasmonic acid production according to the present embodiment can be referred to as a resistance-inducing agent for plants. The resistance-inducing effects include enhancement of resistance to pathogen infection, enhancement of resistance to insect damage, etc.

It is also known that jasmonic acid has growth-modulating effects on plants. Therefore, the agent for promoting endogenous jasmonic acid production according to the present embodiment can be referred to as a growth-modulating agent for plants. The growth-modulating effects include, for example, promotion of rooting, promotion of germination, promotion of flower setting, promotion of flowering, promotion of growth, improvement of fruiting rate, enlargement of fruit, ripening stage promotion, improvement of coloration, improvement of sugar content, and prevention of fruit drop.

Incidentally, whether or not the endogenous jasmonic acid production in plants has been promoted can be confirmed by directly measuring the amount of jasmonic acid produced and also by measuring the expression level of genes that respond to jasmonic acid signals (jasmonic acid responsive genes). The jasmonic acid responsive genes include PDF1.2 gene, PR-4 gene, PR-1b gene, and VSP2 gene, and the like.

The agent for promoting endogenous jasmonic acid production according to the present embodiment may contain, in addition to the compound represented by the formula (1) or a salt thereof, other component such as an agrochemically acceptable carrier (a solid carrier, a liquid carrier, or a gas carrier), a surfactant, a stabilizing agent, and other formulation aid.

Examples of the solid carrier include: minerals such as kaolinite, attapulgite, bentonite, montmorillonite, pyrophyllite, sericite, talc, acid clay, and diatomaceous earth; vegetable organic substances such as corn cob flour, walnut shell flour, wheat flour, soybean flour, and wood flour; and synthetic polymer compounds such as coumarone resin, petroleum resin, alkyd resin, polyvinyl chloride, and ketone resin; and the like.

Examples of the liquid carrier include: water; alcohols such as methanol, ethanol, and isopropanol; ketones such as acetone, ethyl methyl ketone, and cyclohexanone; ethers such as diethyl ether, dioxane, and tetrahydrofuran; esters such as ethyl acetate, amyl acetate, and ethylene glycol acetate; aromatic hydrocarbons such as benzene, toluene, and methylnaphthalene; aliphatic hydrocarbons such as n-hexane, kerosene, and white oil; halogenated hydrocarbons such as dichloroethane, and carbon tetrachloride; vegetable oils such as soybean oil, and cotton seed oil; acid amides such as dimethylformamide, and dimethylacetamide; nitriles such as acetonitrile, and isobutyronitrile; sulfoxides such as dimethyl sulfoxide; and the like.

Examples of the gas carrier include liquefied petroleum gas (LPG), air, a nitrogen gas, a carbon dioxide gas, dimethyl ether, and the like.

Examples of the surfactant include alkyl sulfate esters, alkylsulfonate salts, alkylarylsulfonate salts, polyoxyalkylene alkyl ethers, polyhydric alcohol esters, lignosulfonate salts, and the like.

Examples of the stabilizing agent include acidic isopropyl phosphate (PAP), tricresyl phosphate (TCP), and the like.

Examples of the formulation aid include casein, gelatin, carboxymethyl cellulose, gum arabic, polyethylene glycol, calcium stearate, and the like.

The agent for promoting endogenous jasmonic acid production according to the present embodiment may further contain, in addition to those described above, a phytohormone, a fungicide, an insecticide, a herbicide, a fertilizer, or the like.

The dosage form of the agent for promoting endogenous jasmonic acid production according to the present embodiment is not particularly limited. Examples of the dosage form of the agent for promoting endogenous jasmonic acid production include powders, granules, dust-granule mixture, wettable powders, tablets, water soluble powders, emulsions, suspensions, solutions, oils, pastes, aerosols, and the like.

The agent for promoting endogenous jasmonic acid production according to the present embodiment may be used directly without dilution, or may be used with dilution, if necessary.

### <Method for Promoting Endogenous Jasmonic Acid Production>

A method for promoting endogenous jasmonic acid production according to the present embodiment includes contacting the aforementioned agent for promoting endogenous jasmonic acid production according to the present embodiment with a plant. The contact of the agent for promoting endogenous jasmonic acid production according to the present embodiment with the plant allows for the promotion of the endogenous jasmonic acid production in the plant.

As described above, jasmonic acid is known to have resistance-inducing effects and growth-modulating effects in plants. Therefore, the method for promoting endogenous jasmonic acid production according to the present embodiment can be referred to as a method for inducing resistance in plants or a method for modulating the growth of plants.

Examples of the target plant include cereals (rice, wheat, etc.), beans (soybeans, adzuki beans (red beans), etc.), tubers and roots (potatoes, sweet potatoes, etc.), fruits (apples, grapes, etc.), leafy vegetables (cabbages, spinach, etc.), fruit vegetables (tomatoes, eggplants, etc.), root vegetables (radishes, carrots, etc.), flowers and ornamental plants (chrysanthemums, roses, etc.), specialty crops (cottons, hemp, etc.), and the like.

The part of the plant for application is not particularly limited, and may be appropriately selected according to the type of the plant, the purpose of the application, and the like. Examples of the part for application include seeds, flower buds, flowers (flower clusters), fruits (fruit clusters), stems and leaves, and roots.

The method for application to plants is not particularly limited, and can be appropriately selected according to the type of plant, the dosage form of the agent for promoting endogenous jasmonic acid production, and the purpose of the application, and the like. Examples of the application method include soaking of seeds or coating on seeds; spraying or sprinkling on stems and leaves; irrigating or spraying on the soil; addition to hydroponic solution; and the like. The application to the plant may be done only once or multiple times.

### EXAMPLES

Hereinafter, the present invention will be explained in more detail by way of examples, but the present invention is not limited by these examples.

It is to be noted that in the following Test Examples 1 and 2, compounds E1 and E2 represented by the following formula (each from Maybridge), and compound E3 (from FUJIFILM Wako Pure Chemical Corporation Co. Ltd.) were used as the compound represented by the formula (1). In addition, comparative compound C1 (from Tokyo Chemical Industry Co., Ltd.) and comparative compound C2 (from Sigma-Aldrich), which are represented by the following formulas, were used as compounds for comparison.

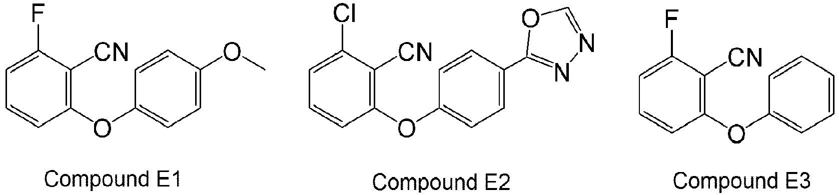

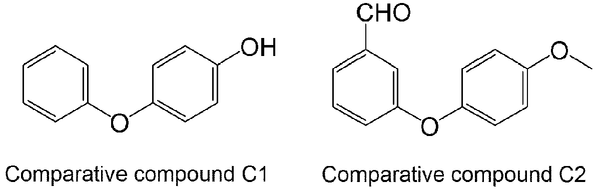

Further, in the following Test Examples 3 to 5, compounds E1, and E4 to E6, which were synthesized in Synthesis Examples 1 to 4, were used as the compound represented by the formula (1) .

### <Synthesis Example 1: Synthesis of Compound E1>

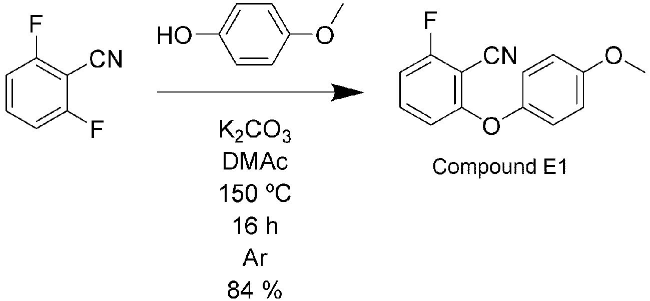

Into an eggplant-type flask were charged 2,6-difluorobenzonitrile (995 mg, 7.16 mmol), 4-methoxyphenol (871 mg, 7.02 mmol), and potassium carbonate (1.401 g, 10.1 mmol), and the flask was flushed with argon and then sealed. The mixture was stirred in dimethylacetamide at 150°C for 16 h. Ethyl acetate was added to the reaction solution to dilute it, and water was added to terminate the reaction. Thereafter, the organic layer was washed once with aqueous saturated ammonium chloride solution and once with saturated saline, followed by washing twice with water. This organic layer was dried over sodium sulfate, and concentrated to give a crude product. The resulting crude product was purified by column chromatography (hexane/ethyl acetate = 10/1), to give compound E1 (1.43 g, 84%) .
¹H NMR (CDCl₃, 400 MHz) δ 7.46-7.40 (m, 1H), 7.32 (t, J = 8.1 Hz, 1H), 6.88 (t, J = 8.1 Hz, 1H), 6.80 (dd, J = 8.1, 2.4 Hz, 1H), 6.69 (t, J = 8.1 Hz, 1H), 6.66 (t, J = 8.1 Hz, 1H), 3.81 (s, 3H).

### <Synthesis Example 2: Synthesis of Compound E4>

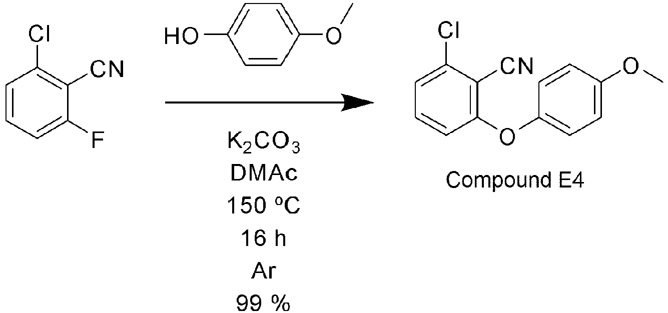

Into an eggplant-type flask were charged 2-chloro-6-fluorobenzonitrile (100.4 mg, 0.6454 mmol), 4-methoxyphenol (79.9 mg, 0.644 mmol), and potassium carbonate (118 g, 0.853 mmol), and the flask was flushed with argon and then sealed. The mixture was stirred in dimethylacetamide at 150°C for 16 h. Ethyl acetate was added to the reaction solution to dilute it, and water was added to terminate the reaction. Thereafter, the organic layer was washed once with aqueous saturated ammonium chloride solution and once with saturated saline, followed by washing twice with water. This organic layer was dried over sodium sulfate, and concentrated to give a crude product. The resulting crude product was purified by column chromatography (hexane/ethyl acetate = 10/1), to give compound E4 (165 mg, 99%).
¹H NMR (CDCl₃, 400 MHz) δ 7.37 (t, J = 8.4 Hz, 1H), 7.13 (d, J = 8.4 Hz, 1H), 7.02 (d, J = 8.6 Hz, 2H), 6.92 (d, J = 8.6 Hz, 2H), 6.66 (d, J = 8.4 Hz, 1H), 3.82 (s, 3H).

### <Synthesis Example 3: Synthesis of Compound E5>

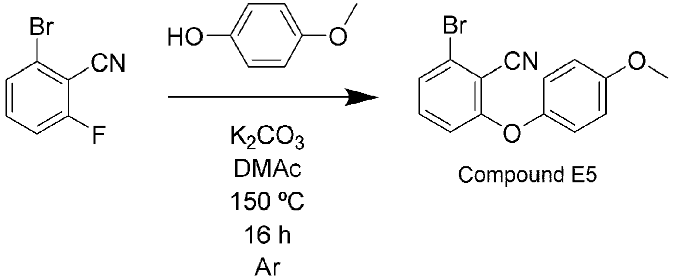

Into an eggplant-type flask were charged 2-bromo-6-fluorobenzonitrile (102 mg, 0.510 mmol), 4-methoxyphenol (63.4 mg, 0.511 mmol), and potassium carbonate (95.6 mg, 0.692 mmol), and the flask was flushed with argon and then sealed. The mixture was stirred in dimethylacetamide at 150°C for 16 h. Ethyl acetate was added to the reaction solution to dilute it, and water was added to terminate the reaction. Thereafter, the organic layer was washed once with aqueous saturated ammonium chloride solution and once with saturated saline, followed by washing twice with water. This organic layer was dried over sodium sulfate, and concentrated to give a crude product. The resulting crude product was purified by column chromatography three times (hexane/ethyl acetate = 10/1, twice; toluene/hexane = 1/2, once), to give compound E5 (134 mg) .
¹H NMR (CDCl₃, 400 MHz) δ 7.31-7.28 (m, 2H), 7.03 (dd, J = 6.5, 2.3 Hz, 2H), 6.93 (dd, J = 8.6 Hz, 2H), 6.70 (dd, J = 8.6 Hz, 1H), 6.69 (t, J = 8.1 Hz, 1H), 3.82 (s, 3H).

### <Synthesis Example 4: Synthesis of Compound E6>

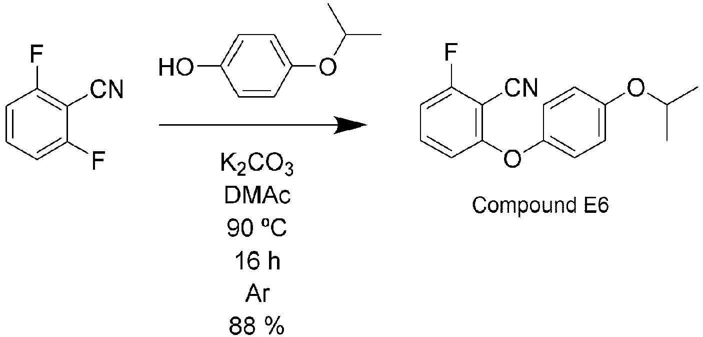

Into an eggplant-type flask were charged 2,6-difluorobenzonitrile (130 mg, 0.934 mmol), 4-isopropoxyphenol (66.8 mg, 0.439 mmol), and potassium carbonate (163 mg, 1.182 mmol), and the flask was flushed with argon and then sealed. The mixture was stirred in dimethylacetamide at 90°C for 16 h. Ethyl acetate was added to the reaction solution to dilute it, and water was added to terminate the reaction. Thereafter, the organic layer was washed once with aqueous saturated ammonium chloride solution and once with saturated saline, followed by washing twice with water. This organic layer was dried over sodium sulfate, and concentrated to give a crude product. The resulting crude product was purified by column chromatography twice (toluene/ethyl acetate = 10/1, once; hexane/ethyl acetate = 10/1, once), to give compound E6 (104 mg, 88%).
¹H NMR (CDCl₃, 400 MHz) δ 7.43-7.38 (m, 1H), 7.02 (d, J = 8.6 Hz, 2H), 6.92 (d, J = 8.6 Hz, 2H), 6.84 (t, J = 8.4 Hz, 1H), 6.56 (d, J = 8.4 Hz, 1H), 4.49 (quin, J = 6.0 Hz, 1H), 1.35 (d, J = 6.0, 6H).

### <Synthesis Example 5: Synthesis of Compound E7>

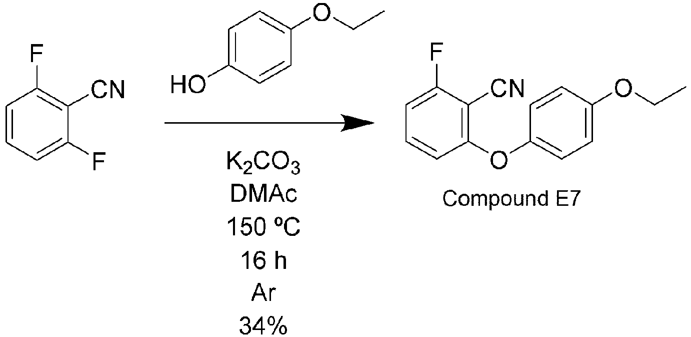

Into an eggplant-type flask were charged 2,6-difluorobenzonitrile (101 mg, 0.727 mmol), 4-ethoxyphenol (100 mg, 0.727 mmol), and potassium carbonate (175.7 mg, 1.2713 mmol), and the flask was flushed with argon and then sealed. The mixture was stirred in dimethylacetamide at 150°C for 16 h. Ethyl acetate was added to the reaction solution to dilute it, and water was added to terminate the reaction. Thereafter, the organic layer was washed once with aqueous saturated ammonium chloride solution and once with saturated saline, followed by washing twice with water. This organic layer was dried over sodium sulfate, and concentrated to give a crude product. The resulting crude product was purified by column chromatography (hexane/ethyl acetate = 10/1), to give compound E7 (63.3 mg, 34%).
¹H NMR (CDCl₃, 400 MHz) δ 7.40-7.36 (m, 1H), 7.03 (d, J = 8.6 Hz, 2H), 6.92 (d, J = 8.6 Hz, 2H), 6.84 (t, J = 8.4 Hz, 1H), 6.53 (d, J = 8.4 Hz, 1H), 4.04 (q, J = 7.0 Hz, 2H), 1.44 (t, J = 7.0, 3H).

### <Synthesis Example 6: Synthesis of Compound E8>

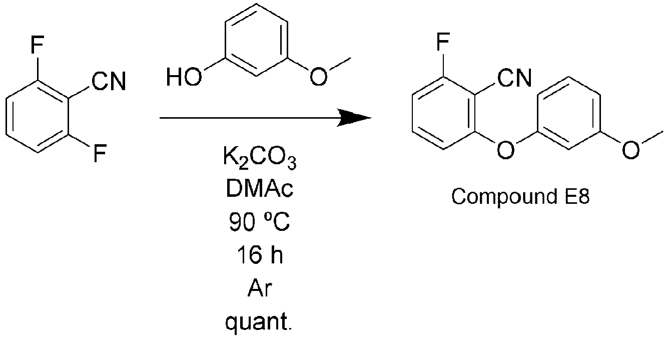

Into an eggplant-type flask were charged 2,6-difluorobenzonitrile (98.3 mg, 0.707 mmol), 3-methoxyphenol (44.6 mg, 0.359 mmol), and potassium carbonate (131 mg, 0.946 mmol), and the flask was flushed with argon and then sealed. The mixture was stirred in dimethylacetamide at 90°C for 16 h. Ethyl acetate was added to the reaction solution to dilute it, and water was added to terminate the reaction. Thereafter, the organic layer was washed once with aqueous saturated ammonium chloride solution and once with saturated saline, followed by washing twice with water. This organic layer was dried over sodium sulfate, and concentrated to give a crude product. The resulting crude product was purified by column chromatography (hexane/ethyl acetate = 10/1), to give compound E8 (95.9 mg, 100%) .
¹H NMR (CDCl₃, 400 MHz) δ 7.47-7.41 (m, 1H), 7.32 (t, J = 8.4 Hz, 1H), 6.88 (t, J = 8.4 Hz, 1H), 6.80 (ddd, J = 0.8, 2.4, 8.4 Hz, 1H), 6.69-6.65 (m, 3H), 3.81 (s, 3H).

### <Synthesis Example 7: Synthesis of Compound E9>

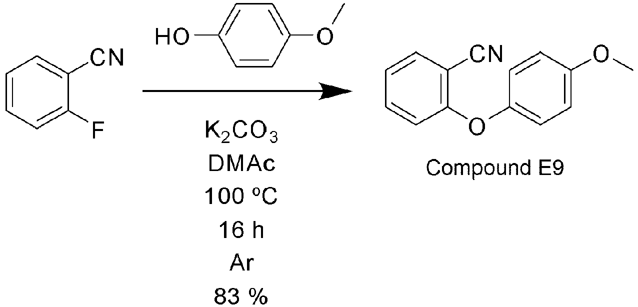

Into an eggplant-type flask were charged 2-fluorobenzonitrile (100 mg, 0.826 mmol), 4-methoxyphenol (102.2 mg, 0.824 mmol), and potassium carbonate (151 mg, 1.09 mmol), and the flask was flushed with argon and then sealed. The mixture was stirred in dimethylacetamide at 100°C for 16 h. Ethyl acetate was added to the reaction solution to dilute it, and water was added to terminate the reaction. Thereafter, the organic layer was washed once with aqueous saturated ammonium chloride solution and once with saturated saline, followed by washing twice with water. This organic layer was dried over sodium sulfate, and concentrated to give a crude product. The resulting crude product was purified by column chromatography twice (hexane/ethyl acetate = 10/1, once; hexane/ethyl acetate = 20/1, once), to give compound E9 (154 mg, 83%).
¹H NMR (CDCl₃, 400 MHz) δ 7.62 (dd, J = 7.7, 1.6 Hz, 1H), 7.46-7.41 (m, 1H), 7.10-7.01 (m, 3H), 6.97-6.90 (m, 2H), 6.77 (d, J = 8.6 Hz, 1H), 3.82 (s, 3H).

### <Synthesis Example 8: Synthesis of Compound E10>

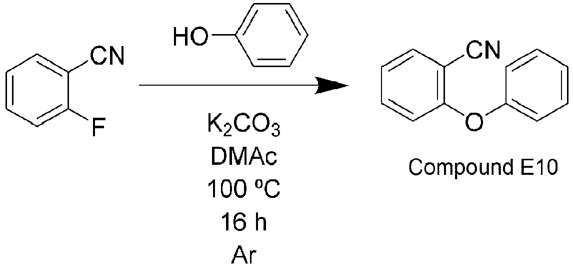

Into an eggplant-type flask were charged 2-fluorobenzonitrile (103 mg, 0.853 mmol), phenol (80.3 mg, 0.853 mmol), and potassium carbonate (158 mg, 1.14 mmol), and the flask was flushed with argon and then sealed. The mixture was stirred in dimethylacetamide at 100°C for 16 h. Ethyl acetate was added to the reaction solution to dilute it, and water was added to terminate the reaction. Thereafter, the organic layer was washed once with aqueous saturated ammonium chloride solution and once with saturated saline, followed by washing twice with water. This organic layer was dried over sodium sulfate, and concentrated to give a crude product. The resulting crude product was purified by column chromatography twice (hexane/ethyl acetate = 20/1), to give compound E10 (148 mg) .
¹H NMR (CDCl₃, 400 MHz) δ 7.65 (dd, J = 7.7, 1.6 Hz, 1H), 7.49-7.46 (m, 1H), 7.45-7.38 (m, 2H), 7.26-7.20 (m, 1H), 7.15-7.07 (m, 3H), 6.85 (dd, J = 8.5, 0.6 Hz, 1H).

### <Test Example 1: Measurement of Amount of Endogenous Jasmonic Acid Production>

### (Sample Preparation)

*Arabidopsis thaliana* (Col-0) seeds were sown on a transparent 96-well plate, and soaked in a 1/2 MS medium (150 µL). The seeds were left for 2-3 days in a cool and dark place, and then left to grow under long-day treatment conditions for 9-10 days. Thereafter, the medium was replaced with a 1/2 MS medium (100 µL) containing each (50 µM) of compounds E1, E2, and E3, and comparative compounds C1 and C2, vacuum treatment was applied for 10 min, and the seeds were left in the light for 24 h. About 20 to 60 mg of the plantlets (about 3 individuals) and 2 beads for crushing were placed in a tube, and immediately frozen with liquid nitrogen and stored at -80°C. In addition, for a negative control, DMSO was added instead of each of compounds E1, E2, and E3, and comparative compounds C1 and C2.

### (Extraction and Measurement of Jasmonic Acid)

The frozen sample was crushed using a bead-type homogenizer (Bead Smash 12; WakenBtech Co., Ltd.) under conditions of 3,000 rpm for 60 sec, and then 1 mL of an aqueous solution of 70% methanol plus 1% acetic acid and 10 ng of d2-JA (internal standard) were added. The mixture was mixed thoroughly by vortexing. This solution was centrifuged at 10,000 rpm for 5 min and the supernatant was collected. The same process was repeated two more times without the addition of the internal standard, and a total of 3 mL of an extract was collected.

The solutions were then applied to a Sep-Pak (registered trademark) Vac 3cc (500 mg) C18 Cartridge (Waters) in the following order of 1. to 6.
1. acetonitrile (one column volume, about 3 mL)
2. 100% methanol (one column volume)
3. distilled water (one column volume)
4. aqueous solution of 70% methanol + 1% acetic acid (two column volumes)
5. sample supernatant (total amount)
6. aqueous solution of 70% methanol + 1% acetic acid (one column volume)

Then, a solution eluted from immediately after the application in 5. above until the completion of the elution of 6. above was collected (about 6 mL), and the collected solution was concentrated to 30% or less of its original volume by nitrogen blowing method.

Then, the solutions were applied to an Oasis (registered trademark) MAX 3cc (60 mg) Extraction Cartridge (Waters) in the following order 1. to 11.
1. acetonitrile (one column volume, about 3 mL)
2. 100% methanol (one column volume)
3. 1% aqueous acetic acid solution (one column volume)
4. concentrated sample (one column volume)
5. 50 mM potassium phosphate buffer (pH 8.0) (one column volume)
6. distilled water (one column volume)
7. 100% methanol (one column volume)
8. distilled water (one column volume)
9. 1% aqueous acetic acid solution (one column volume)
10. aqueous solution of 30% methanol + 1% acetic acid (one column volume)
11. aqueous solution of 70% methanol + 1% acetic acid (two column volumes)

Then, a solution eluted from immediately after the application in 11. above until the completion of the elution of 11. was collected (about 6 mL), and the collected solution was concentrated to 1 mL or less by nitrogen blowing method to prepare a measurement sample. The measurement sample was dispensed into a measurement tube of a liquid chromatography tandem mass spectrometer (LC-MS/MS), and then stored at -80°C until measurement.

Then, the amount of endogenous jasmonic acid (JA) production was determined by LC-MS/MS. The determination conditions are as follows. In addition, the detection conditions are shown in Table 1 below.
Mobile layer: acetonitrile/water (containing 0.1% acetic acid) = 3/7
flow rate: 0.2 mL/min
retention time: 10.9 to 11.1 min
amount of injected sample: 10 µL
column: C8/ 2 mm × 100 mm

**[Table 1]**

| | JA | d2-JA |
|---|---|---|
| Precursor m/z | 209.20 | 211.20 |
| Product m/z | 59.00 | 59.10 |
| Dwell Time | 100.0 msec | 100.0 msec |
| Q1 Pre Bias | 19.0 V | 11.0 V |
| CE (Collision energy) | 12.0 | 14.0 |
| Q3 Pre Bias | 19.0 V | 21.0 V |

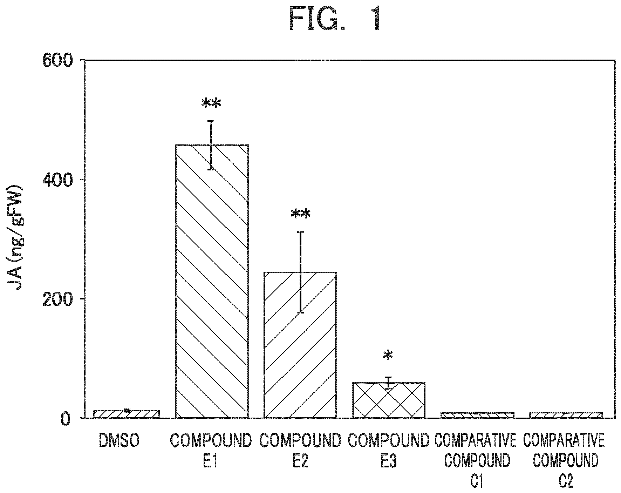

The amount of endogenous jasmonic acid production in *Arabidopsis thaliana* contacted with each of compounds E1, E2, and E3, and comparative compounds C1 and C2 is shown in FIG. 1. As can be seen from FIG. 1, the amount of endogenous jasmonic acid production was significantly increased when *Arabidopsis thaliana* was contacted with compound E1, E2, or E3 (*p < 0.05; **p < 0.01).

### <Test Example 2: Measurement of Expression Level of PDF1.2 Gene (1)>

### (Sample Preparation)

*Arabidopsis thaliana* (Col-0) seeds were sown on a transparent 96-well plate, and soaked in a 1/2 MS medium (150 µL). The seeds were left for 2-3 days in a cool and dark place, and then left to grow under long-day treatment conditions for 9-10 days. Thereafter, the medium was replaced with a 1/2 MS medium (100 µL) containing compound E1 (25 µM) or compound E2 (50 µM), vacuum treatment was applied for 10 min, and the seeds were left in the light for 24 h. About 20 to 60 mg of the plantlets (about 3 individuals) and 2 beads for crushing were placed in a tube, and immediately frozen with liquid nitrogen and stored at -80°C. In addition, for a negative control, DMSO was added instead of each of compounds E1 and E2.

### (RNA Extraction and Reverse Transcription Reaction)

The frozen sample was crushed using a bead-type homogenizer (Bead Smash 12; WakenBtech Co., Ltd.) under conditions of 3,000 rpm for 60 sec, and then RNA extraction was performed using NucleoSpin RNA Plus (Takara Bio Inc.).

Then, a reverse transcription reaction was performed using Rever Tra Ace qPCR RT Master Mix with gDNA Remover (Toyobo Co., Ltd.). The concentration of the total RNA obtained was measured, and the total RNA was mixed with RNAse-free H₂O to 1/12 µg/µL (6 µL H₂O for 0.5 µg of the total RNA), and the mixture was placed in a PCR tube, incubated at 65°C for 5 min, and then quenched on ice. Then, 4×DN Master Mix (2 µL) was added to the quenched mixture, and the total mixture was subjected to tapping, centrifuged, and incubated at 37°C for 5 min. After the incubation, 5×RT Master Mix II (2 µL) was added, and the mixture was incubated sequentially at 37°C for 15 min, at 50°C for 5 min, and then at 98°C for 5 min, and the resulting sample was used as a cDNA sample.

### (Real-Time PCR)

Sterile water was added to the cDNA sample to dilute it 8 to 10 times. The diluted sample (1 µL), distilled water (3.4 µL), various primers (10 µM) (0.3 µL for each of the forward and reverse primers), and THUNDERBIRD SYBR qPCR Mix (Toyobo Co., Ltd.) (5 µL) were mixed, and the mixture was dispensed into one well of a 96-well plate for real-time PCR.

Then, CFX Connect Real-Time PCR Detection System (Bio-Rad) was used to quantitatively determine the expression level of PDF1.2 gene. The PCR conditions were [95°C, 60 sec] × 1 cycle, [95°C, 15 sec → 60°C, 60 sec] × 40 cycles, and [95°C, 15 sec → 60°C, 30 sec → 95°C, 15 sec] × 1 cycle. For quantification, the ΔΔCt method was used, and this value was expressed as the relative expression level. EF1-α gene was used as the reference gene for the ΔΔCt method. The sequences of the primers used in the real-time PCR are shown in Table 2 below.

**[Table 2]**

| Gene symbol | Primer sequence | SEQ ID No. |
|---|---|---|
| *PDF1.2* | Forward 5'-TCATGGCTAAGTTTGCTTCC-3' | 1 |
| | Reverse 5'-AATACACACGATTTAGCACC-3' | 2 |
| *EF1-α* | Forward 5'-TGAGCACGCTCTTCTTGCTTTCA-3' | 3 |
| | Reverse 5'-GGTGGTGGCATCCATCTTGTTACA-3' | 4 |

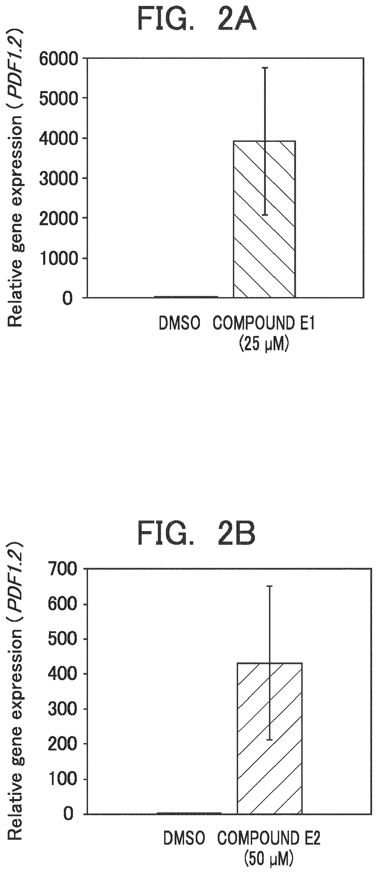

The expression levels of the PDF1.2 gene in *Arabidopsis thaliana* contacted with each of compounds E1 and E2 are shown in FIGs. 2A and 2B. As can be seen from FIGs. 2A and 2B, the expression of the PDF1.2 gene was markedly enhanced when *Arabidopsis thaliana* was contacted with compound E1 or E2.

### <Test Example 3: Measurement of Expression Level of PDF1.2 Gene (2)>

*Arabidopsis thaliana* (Col-0) seeds were sown on a transparent 96-well plate, and soaked in a 1/2 MS medium (150 µL). The seeds were left for 2-3 days in a cool and dark place, and then left to grow under long-day treatment conditions for 10 days. Thereafter, the medium was replaced with a 1/2 MS medium (100 µL) containing compound E1 (50 µM), compound E4 (50 µM), or compound E5 (50 µM), and comparative compounds C1 and C2, vacuum treatment was applied for 10 min, and the seeds were left in the light for 48 h. Eight individual plantlets and 2 beads for crushing were placed in a tube, and immediately frozen with liquid nitrogen and stored at -80°C. In addition, for a negative control, DMSO was added instead of each of compounds E1, E4, and E5. Then, similarly to Test Example 2, RNA was extracted from the frozen sample, and the expression level of the PDF1.2 gene was quantitatively determined by reverse transcription real-time PCR.

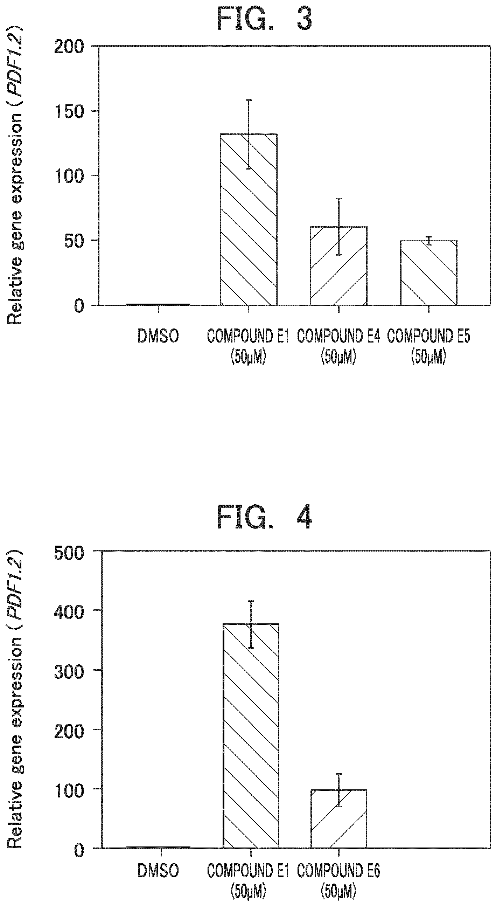

The expression levels of PDF1.2 gene in *Arabidopsis thaliana* contacted with each of compounds E1, E4, and E5 are shown in FIG. 3. As can be seen from FIG. 3, the expression of the PDF1.2 gene was markedly enhanced when *Arabidopsis thaliana* was contacted with compound E1, E4, or E5.

### <Test Example 4: Measurement of Expression Level of PDF1.2 Gene (3)>

*Arabidopsis thaliana* (Col-0) seeds were sown on a transparent 96-well plate, and soaked in a 1/2 MS medium (150 µL). The seeds were left for 3-7 days in a cool and dark place, and then left to grow under long-day treatment conditions for 10 days. Thereafter, the medium was replaced with a 1/2 MS medium (100 µL) containing compound E1 (50 µM), or compound E6 (50 µM), and comparative compounds C1 and C2, vacuum treatment was applied for 10 min, and the seeds were left in the light for 48 h. Eight individual plantlets and 2 beads for crushing were placed in a tube, and immediately frozen with liquid nitrogen and stored at -80°C. In addition, for a negative control, DMSO was added instead of each of compounds E1 and E6. Then, similarly to Test Example 2, RNA was extracted from the frozen sample, and the expression level of the PDF1.2 gene was quantitatively determined by reverse transcription real-time PCR.

The expression levels of the PDF1.2 gene in *Arabidopsis thaliana* contacted with each of compounds E1 and E6 are shown in FIG. 4. As can be seen from FIG. 4, the expression of PDF1.2 gene was markedly enhanced when *Arabidopsis thaliana* was contacted with compound E1 or E6.

### <Test Example 5: Study on Cytotoxicity of Compound E1>

Normal fibroblasts MRC-5 from human fetal lung were seeded in a 96-well plate at a cell density of 5,000 cells/well, and cultured for 1 day under conditions of 37°C and 5% CO₂. After the culturing, compound E1 was added to the medium at varying concentrations, and the cells were cultured for another 2 days. Then, the number of viable cells was determined using the WST-8 method, and the viability of the MRC-5 cells was calculated. In addition, for a control, SN-38, an active metabolite of the anticancer drug irinotecan, was added instead of compound E1.

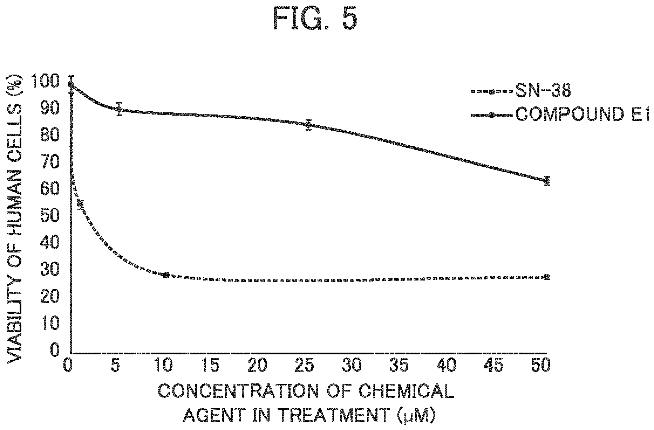

The viability of the MRC-5 cells is shown in FIG. 5. As shown in FIG. 5, the viability of the MRC-5 cells exceeds 60% even in the case where compound E1 at 50 µM was added, indicating low cytotoxicity.

## Claims

1. An agent for promoting endogenous jasmonic acid production comprising a compound represented by formula (1) or a salt thereof as an active ingredient, 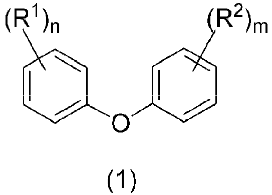
wherein in the formula (1), R¹ and R² represent, each independently, a monovalent organic group, a cyano group, a halogen atom, a hydroxy group, an amino group, a nitro group, a nitroxy group, a mercapto group, a cyanate group, a thiocyanate group, an isothiocyanate group, a sulfo group, a sulfamino group, a sulfino group, a sulfamoyl group, a phospho group, a phosphono group, or a boronyl group, wherein n R¹ comprise at least a cyano group or a halogen atom;
n represents an integer of 1 to 5,
wherein in a case where n is 2 or more, n R¹ are identical to or different from one another, and wherein two or more R¹ bonded to adjacent carbon atoms optionally bond to one another to form a ring structure; and
m represents an integer of 0 to 5,
wherein in a case where m is 2 or more, m R² are identical to or different from one another, and wherein two or more R² bonded to adjacent carbon atoms optionally bond to one another to form a ring structure.

2. The agent for promoting endogenous jasmonic acid production according to claim 1, wherein n R¹ comprise at least a cyano group.

3. The agent for promoting endogenous jasmonic acid production according to claim 1 or 2, wherein n is 2 or more.

4. The agent for promoting endogenous jasmonic acid production according to claim 3, wherein n R¹ comprise at least a cyano group and a halogen atom.

5. The agent for promoting endogenous jasmonic acid production according to claim 4, wherein the compound represented by the formula (1) is a compound represented by formula (2): 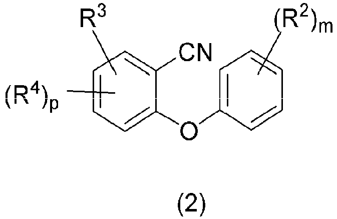
wherein in the formula (2), R³ represents a halogen atom; R⁴ represents a monovalent organic group, a cyano group, a halogen atom, a hydroxy group, an amino group, a nitro group, a nitroxy group, a mercapto group, a cyanate group, a thiocyanate group, an isothiocyanate group, a sulfo group, a sulfamino group, a sulfino group, a sulfamoyl group, a phospho group, a phosphono group, or a boronyl group;
p represents an integer of 0 to 3,
wherein in a case where p is 2 or more, p R⁴ are identical to or different from one another, and wherein two or more R⁴ bonded to adjacent carbon atoms optionally bond to one another to form a ring structure; and
R² and m are as defined in the formula (1).

6. The agent for promoting endogenous jasmonic acid production according to claim 5, wherein the compound represented by the formula (2) is a compound represented by formula (3-1) or (3-2): 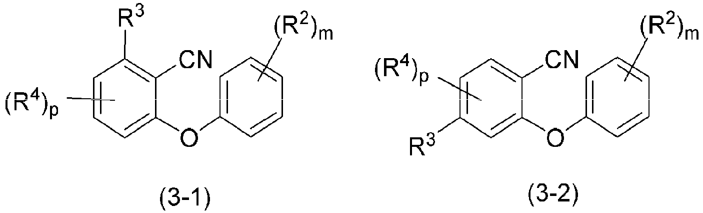 wherein in the formulas (3-1) and (3-2), R², R³, R⁴, m, and p are as defined in the formula (2).

7. A method for promoting endogenous jasmonic acid production, comprising contacting the agent for promoting endogenous jasmonic acid production according to any one of claims 1 to 6 with a plant.
